# FASCICULE DE BREVET EUROPEEN

(11) **EP 1 301 477 B1**
(45) Date de publication et mention de la délivrance du brevet: **29.09.2004**
(21) Numéro de dépôt: 01954111.9
(22) Date de dépôt: 17.07.2001
(51) Int. Cl.: C07C 319/24, C07C 319/02, C07C 323/66

(54) **PROCEDE DE PREPARATION DU 2,2'-DITHIOBIS(ETHANESULFONATE) DE DISODIUM**
VERFAHREN ZUR HERSTELLUNG VON DINATRIUM-2,2'-DITHIOBIS(ETHANSULFONAT)
METHOD FOR PREPARING DISODIUM 2.2'-DITHIOBIS(ETHANESULPHONATE)

(30) Priorité: 18.07.2000 FR 0009386
(43) Date de publication de la demande: 16.04.2003
(73) Titulaire: BIONUMERIK PHARMACEUTICALS, INC., San Antonio, Texas 78229 (US)
(72) Inventeur: CAZAUX, Jean-Bernard, F-30390 Aramon (FR); MANGINOT, Eric, F-84140 Montfavet (FR); VEYRAT, Marc, F-84130 Le Pontet (FR)
(74) Mandataire: Lucas, Brian Ronald
(86) Numéro de dépôt international: PCT/FR2001/002312
(87) Numéro de publication internationale: WO 2002/006216

(56) Documents cités:
- WO-A-98/14426
- US-A- 2 396 879
- DATABASE CHEMABS [en ligne] CHEMICAL ABSTRACTS SERVICE, COLUMBUS, OHIO, US; STN, CAPLUS accession no. 1989:7669, XP002160848 & CS 252 564 A (J. JARY ET AL) 17 septembre 1987 (1987-09-17)

## Description

La présente invention concerne un nouveau procédé de préparation industrielle de 2,2'-dithiobis(aikylsulfonates) de disodium, et en particulier du 2,2'-dithiobis(éthanesulfonate) de disodium (dimesna).

Le dimesna (tout comme son monomère le mesna) est un agent thérapeutique utile entre autres comme agents protecteurs administrés aux patients atteints de certains types de cancer pour diminuer l'effet toxique de certains complexes du platine utilisés en chimiothérapie comme le *cis*-platine (cf. notamment la demande de brevet PCT WO 98/14426).

Un certain nombre de demandes de brevet est consacré à des procédés de préparation du dimesna ou du mesna. Parmi elles, on peut notamment citer la demande de brevet PCT WO 98/14426. Les procédés relatifs à la préparation du dimesna ou de produits apparentés décrits dans cette dernière peuvent être résumés par le schéma réactionnel ci-dessous, dans lequel Hal représente un atome halogène et R₂ représente un radical SO₃M ou PO₃M₂, l'atome M pouvant être le sodium, le potassium ou l'hydrogène, étant entendu que lorsque le réactif de formule générale (**IIb**) est employé, R₂ représente exclusivement un radical PO₃M₂ :

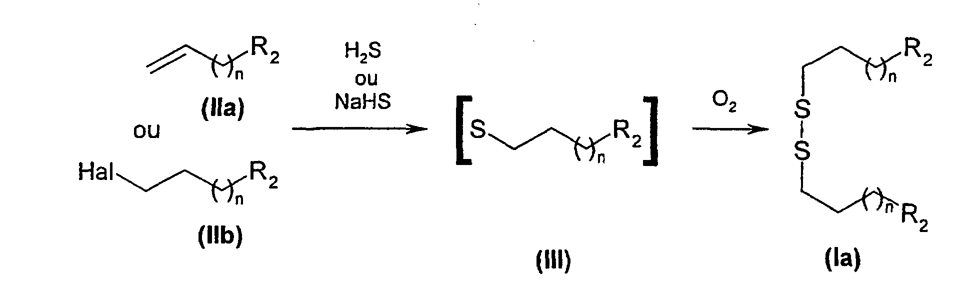

Dans ce procédé, l'intermédiaire de synthèse de formule générale **(III)** n'est pas isolé et conduit directement par chauffage en présence d'oxygène au disulfure de formule générale **(Ia).**

Une autre méthode d'accès aux disulfures symétriques comme le dimesna est décrite dans *Phosphorus, Sulfur and Silicon* (1994), **95-96**, 351-352. Cette méthode peut être résumée par le schéma synthétique suivant, dans lequel (BTS)₂ représente le disulfure du 2-mercaptobenzotriazole :

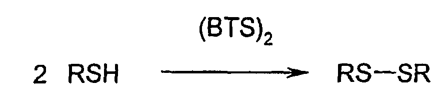

Un problème courant lors de la synthèse du mesna ou du dimesna consiste néanmoins en la présence dans le mesna ou le dimesna obtenu de nombreuses impuretés, généralement des sels comme du bromure de sodium ou des sels de type acétate issus des procédés de préparation employés jusqu'à présent. L'élimination de ces impuretés pour obtenir un produit utilisable dans la préparation du dimesna représente une importante perte de temps et d'argent.

La demanderesse a maintenant mis au point un nouveau procédé qui permet d'obtenir des 2,2'-dithiobis(alkylsulfonates) de disodium, et en particulier du dimesna, sous une forme exempte des impuretés résultant des procédés employés jusqu'à présent.

Ce nouveau procédé de préparation industrielle de 2,2'-dithiobis(alkylsulfonates) de disodium est résumé dans le schéma réactionnel ci-dessous :

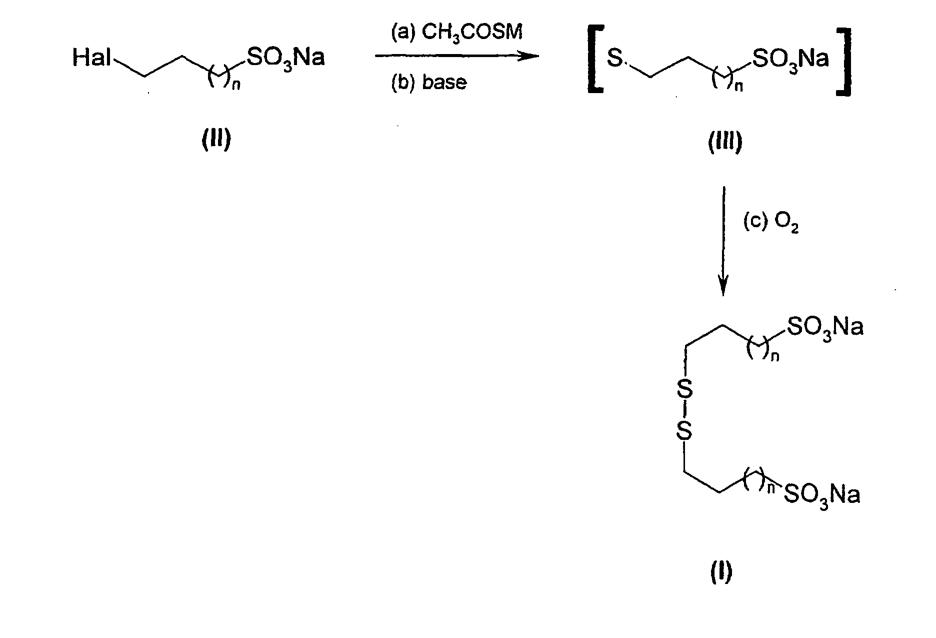

Dans le schéma ci-dessus, Hal représente un atome halogène, et de préférence un atome de brome, M représente un atome de sodium ou de potassium et n représente un entier de 0 à 2. Les étapes (a) et (c) sont de préférence effectuées en chauffant. De préférence, M représente un atome de sodium.

L'invention concerne donc un procédé de préparation industrielle d'un disulfure de formule générale **(I)** 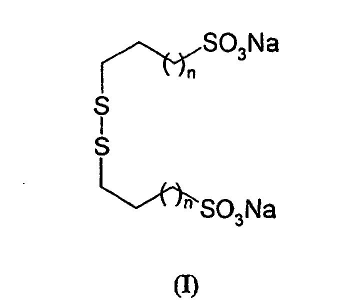 dans laquelle n représente un entier de 0 à 2,
caractérisé en ce qu'il comporte les étapes successives suivantes :
(a) traitement d'un composé de formule générale **(II)** 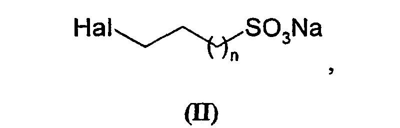 dans laquelle Hal est un atome halogène, avec un thiolacétate de formule générale CH₃COSM dans laquelle M représente un atome de sodium ou de potassium ;
(b) réaction du thiolacétate obtenu à l'étape (a) avec une base suivie d'une neutralisation à l'aide d'un acide ; et
(c) réaction du mercaptoalkylsulfonate de sodium de formule générale (**III**) 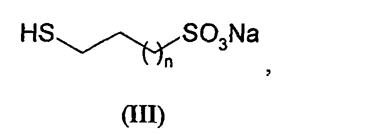 obtenu de façon intermédiaire avec de l'oxygène, pour donner le disulfure de formule générale **(I);**
(d) ajout d'éthanol au mélange réactionnel obtenu à l'étape (c), chauffage afin de solubiliser les précipités puis refroidissement et lavage au moins une fois du solide obtenu avec de l'éthanol.

Le thiolacétate de sodium CH₃COSNa de l'étape (a) du procédé ci-dessus peut être obtenu par réaction d'acide thiolacétique avec une base ayant comme contre-ion le métal M, par exemple NaOH ou KOH lorsque M représente respectivement Na ou K. De préférence, le métal M sera le sodium. De préférence également, la réaction de l'étape (a) se fera en chauffant à une température supérieure à la température ambiante, par exemple à une température comprise entre 25 et 100° C, notamment entre 75 et 85° C.

Dans l'étape (b) du procédé ci-dessus, la base destinée à l'hydrolyse du thioacétate obtenu intermédiairement sera de préférence NaOH ou KOH. Etant donné le caractère exothermique de la réaction, le milieu réactionnel obtenu après l'étape (a) sera de préférence refroidi à une température plus proche de (voire inférieure à) la température ambiante que la température de l'étape (a) (par exemple, si la température préférée pour l'étape (a) était comprise entre 75 et 85° C, on préférera effectuer l'étape (b) avec une température initiale de 45 à 55° C, ou même à une température comprise entre 0 et 45° C). Le pH du milieu réactionnel à l'issue de cette étape sera compris entre 6 et 8, de préférence entre 6,5 et 7,5 et plus préférentiellement entre 7 et 7,2. L'acide employé pour amener le pH à une valeur approximativement neutre sera de préférence l'acide acétique, mais on pourra aussi employer tout acide adapté.

Dans l'étape (c) du procédé ci-dessus, le milieu réactionnel sera de préférence chauffé à une température supérieure à la température ambiante, par exemple à une température comprise entre 30 et 90° C, notamment entre 50 et 60° C. L'oxygène sera laissé buller dans le milieu réactionnel à une pression de préférence comprise entre 0 et 5 bars, et plus préférentiellement à la pression atmosphérique. Alternativement, de l'eau oxygénée en quantité approximativement stoechiométrique pourra être employée à la place d'oxygène gazeux. La réaction s'effectuera de préférence pendant une période de 2 à 10 h et plus, par exemple pendant 8 heures (cette durée étant avant tout déterminée par la durée de réaction dont la fin effective peut, par exemple, être vérifiée par HPLC, chromatographie sur couche mince ou tout autre moyen que l'homme du métier jugera adapté en l'occurrence). A l'issue de l'étape (c), le milieu réactionnel est éventuellement filtré, par exemple sur un filtre de maillage de diamètre compris entre 0,5 et 5 µm, et de préférence de maillage compris entre 0,5 et 1 µm.

Dans l'étape (d) du procédé ci-dessus, le volume d'éthanol ajouté est fonction du volume V du milieu réactionnel obtenu après l'étape (c) et est de préférence compris entre 0,8 et 1,2 fois le volume réactionnel obtenu à l'étape (c), et plus préférentiellement entre 0,9 et 1,1 fois ce même volume. Le mélange obtenu après addition d'éthanol sera chauffé à une température suffisante pour solubiliser les précipités. Cette température pourra être par exemple comprise entre 60 et 70° C. Le refroidissement de la solution ainsi obtenu sera de préférence lent et la solution sera de préférence laissée reposer plusieurs heures à température ambiante. Toujours de préférence, la solution sera ensuite refroidie à une température comprise entre 0 et 40° C, plus préférentiellement entre 0 et 10° C, et sera maintenue au moins 30 minutes, par exemple 1 heure, à cette température. Selon une variante préférée du procédé selon l'invention, le premier lavage est effectué à l'aide d'un mélange éthanol/eau, la proportion dudit mélange étant de préférence supérieure à 2 volumes d'éthanol pour un volume d'eau, et par exemple de 3 volumes d'éthanol pour un volume d'eau.

A l'issue de l'étape (d), le produit du procédé selon l'invention peut être séché en étuve ventilée et l'absence d'éthanol / d'eau résiduelle vérifiée par RMN. Alternativement, ce produit peut être séché sous pression réduite ou selon toute autre méthode adaptée connue de l'homme du métier.

Selon une variante particulièrement préférée de l'invention, le composé de formule générale **(I)** est tel que n = 0 et est donc le dimesna.

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnées ici sont incorporées par référence.

L'exemple suivant est présenté pour illustrer les procédures ci-dessus et ne doit en aucun cas être considéré comme une limite à la portée de l'invention.

### EXEMPLE :

### Préparation du 2,2'-dithiobis(éthanesulfonate) de disodium (dimesna)

*Dans le mode opératoire ci-après, lorsqu'une température est précédée de la mention « environ », elle correspond à un intervalle de température de ± 5° C autour de la température indiquée.*

Dans un premier réacteur R, une solution de soude est préparée par mélange de 2 1 d'eau filtrée et 0,65 kg de lessive de soude (1,03 éq.). La solution obtenue est homogénéisée à environ 5° C. 0,388 kg d'acide thiolacétique (1,08 éq.) sont ajoutés à la solution précédente, la température étant toujours maintenue à environ 5° C. 0,1 l d'eau filtrée sont alors utilisés pour rincer le réservoir d'acide thiolacétique et ajoutés à la solution obtenue. Le mélange du réacteur R est alors réchauffé à environ 20° C et agité pendant 30 minutes à cette température.

Dans un second réacteur R', 1 kg de bromoéthanesulfonate de sodium est chargé (1,16 éq.). La solution du réacteur R est ajoutée au contenu du second réacteur. Le réacteur R est rincé avec 0,11 d'eau filtrée et l'eau de rinçage additionnée au contenu du réacteur R'. Le mélange du réacteur R' est porté à une température d'environ 80° C et agité à cette température pendant environ 1 h 30. La fin effective de la réaction est vérifiée par HPLC. La solution est alors refroidie à environ 50° C.

Dans un autre réacteur, une solution de soude est préparée par mélange de 2 l d'eau filtrée et 1,36 kg de lessive de soude. La solution obtenue est homogénéisée à environ 20° C. Cette solution est coulée dans le réacteur R', provoquant une réaction exothermique. Le milieu réactionnel est agité pendant environ 30 min. La fin effective de la réaction est vérifiée par HPLC. Le mélange du réacteur R'est alors amené à une température d'environ 55° C.

Toujours dans un autre réacteur, une solution d'acide acétique est préparée par mélange de 0,3 l d'eau filtrée et 0,3 l d'acide acétique. Cette solution est coulée dans le réacteur R'. Une fois la vérification faite que le pH est bien compris entre 7,0 et 7,2, de l'oxygène est bullé dans le milieu réactionnel, la température du mélange ainsi obtenu dans le réacteur R' étant constamment maintenue à environ 55° C. Après 8 h de contact, le pH est contrôlé à nouveau et la fin effective de la réaction vérifiée par HPLC (si la réaction n'est pas terminée, on continue à faire buller de l'oxygène à une température d'environ 55° C aussi longtemps que nécessaire). Le milieu réactionnel est alors filtré sur filtre de maillage environ 1 µm.

De l'éthanol est alors ajouté au mélange obtenu après filtration en un volume identique au volume du milieu réactionnel et agité vigoureusement. Des précipités se forment éventuellement, lesquels sont dans ce cas solubilisés par chauffage à une température d'environ 65° C. Le mélange est alors refroidi progressivement à environ 20° C et peut être laissé, le cas échéant, reposer une nuit à 20° C. Le mélange obtenu est alors refroidi à environ 5° C et maintenu durant 1 h à cette température. La suspension est ensuite essorée.

Dans un réacteur à part, on prépare une solution éthanolique par mélange de 0,1875 1 d'eau filtrée et de 0,5625 l d'éthanol. Cette solution, homogénéisée à une température d'environ 20° C, est utilisée pour laver le « gâteau » obtenu par essorage de la suspension précédente. Le « gâteau » est encore lavé avec 0,75 l d'éthanol puis le solide est séché en étuve ventilée à 60° C jusqu'à ce que la RMN ne montre plus la présence d'éthanol. 0,70 kg de dimesna sont alors récupérés.

## Revendications

1. Procédé de préparation industrielle d'un disulfure de formule générale (I) 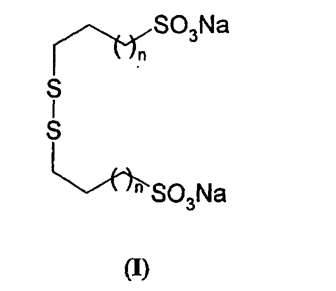 dans laquelle n représente un entier de 0 à 2,
**caractérisé en ce qu'**il comporte les étapes successives suivantes :
(a) traitement d'un composé de formule générale **(II)** 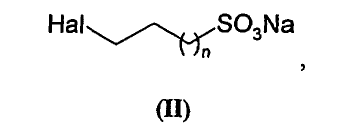 dans laquelle Hal est un atome halogène, avec un thiolacétate de formule générale CH₃COSM dans laquelle M représente un atome de sodium ou de potassium ;
(b) réaction du thiolacétate obtenu à l'étape (a) avec une base suivie d'une neutralisation à l'aide d'un acide ; et
(c) réaction du mercaptoalkylsulfonate de sodium de formule générale **(III)** 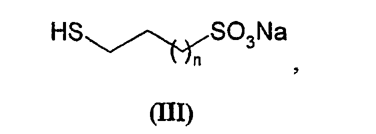 obtenu de façon intermédiaire avec de l'oxygène, pour donner le disulfure de formule générale (I) ;
(d) ajout d'éthanol au mélange réactionnel obtenu à l'étape (c), chauffage afin de solubiliser les précipités puis refroidissement et lavage au moins une fois du solide obtenu avec de l'éthanol.

2. Procédé selon la revendication 1, **caractérisé en ce que** M est un atome de sodium dans l'étape (a).

3. Procédé selon la revendication 1 ou 2, **caractérisé en ce que** la base est NaOH dans l'étape (b).

4. Procédé selon l'un des revendications 1 à 3, **caractérisé en ce que** le volume d'éthanol ajouté à l'étape (d) est compris entre 0,8 et 1,2 fois le volume réactionnel obtenu à l'étape (c).

5. Procédé selon la revendication 4, **caractérisé en ce que** le volume d'éthanol ajouté à l'étape (d) est compris entre 0,9 et 1,1 fois le volume réactionnel obtenu à l'étape (c).

6. Procédé selon l'une des revendications précédentes, **caractérisé en ce que** le lavage de l'étape (d) comprend une première étape de lavage avec un mélange éthanol/eau, la proportion dudit mélange étant supérieure à 2 volumes d'éthanol pour un volume d'eau.

7. Procédé selon la revendication 6, **caractérisé en ce que** le lavage de l'étape (d) comprend une première étape de lavage avec un mélange éthanol/eau, la proportion dudit mélange étant de 3 volumes d'éthanol pour un volume d'eau.

8. Procédé selon l'une des revendications précédentes, **caractérisé en ce que** l'étape (c) est suivie d'une filtration du milieu réactionnel sur un filtre de maillage de diamètre compris entre 0,5 et 5 µm, laquelle est effectuée avant l'étape (d).

9. Procédé selon la revendication 8, **caractérisé en ce que** le filtre employé possède un maillage de diamètre compris entre 0,5 et 1 µm.

10. Procédé selon l'une des revendications précédentes, **caractérisé en ce que** n = 0.

## Patentansprüche

1. Verfahren zur industriellen Herstellung eines Disulfids der allgemeinen Formel (**I**) 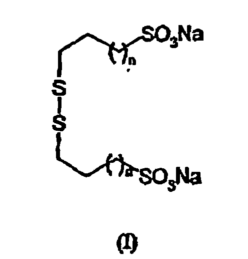 wobei n eine ganze Zahl von 0 bis 2 bedeutet,
**dadurch gekennzeichnet, dass** es die folgenden, aufeinanderfolgenden Schritte umfasst:
(a) Behandlung einer Verbindung der allgemeinen Formel (II) 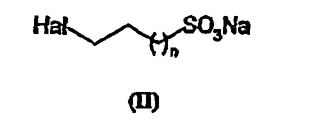 wobei Hal ein Halogenatom ist, mit einem Thiolaceat der allgemeinen Formel CH₃COSM, wobei M ein Natrium- oder Kaliumatom darstellt;
(b) Umsetzung des in Schritt (a) erhaltenen Thiolacetats mit einer Base und anschließende Neutralisation mit einer Säure; und
(c) Umsetzung des intermediär erhaltenen Natriumzinercaptoalksylsulfonats der allgemeinen Formel (III) 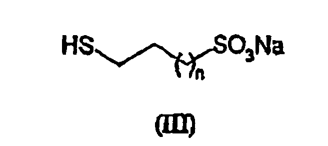 mit Sauerstoff, um das Disulfid der allgemeinen Formel **(I)** zu ergeben;
(d) Zugabe von Ethanol zu dem in Schritt (c) erhaltenen Reaktionsgemisch, Erhitzen, um die Niederschläge zu solubilisieren, anschließend Abkühlen und Waschen des erhaltenen Feststoffs mindestens 1 Mal mit Ethanol.

2. Verfahren nach Anspruch 1, **dadurch gekennzeichnet, dass** M in Schritt (a) ein Natriumatom ist.

3. Verfahren nach Anspruch 1 oder 2, **dadurch gekennzeichnet, dass** die Base in Schritt (b) NaOH ist.

4. Verfahren nach einem der Ansprüche 1 bis 3, **dadurch gekennzeichnet, dass** das in Schritt (d) zugesetzte Volumen an Ethanol dem 0,8- bis 1,2-fachen des in Schritt (c) erhaltenen Reaktionsvolumens entspricht.

5. Verfahren nach Anspruch 4, **dadurch gekennzeichnet, dass** das in Schritt (d) zugesetzte Volumen an Ethanol dem 0,9- bis 1,1-fachen des in Schritt (c) erhaltenen Reaktionsvolumens entspricht.

6. Verfahrea nach einem der vorhergehenden Ansprüche, **dadurch gekennzeichnet, dass** das Waschen in Schritt (d) einen ersten Waschschritt mit einem Ethanol/Wasser-Gemisch umfasst, wobei das Verhältnis des Gemisches größer ist als 2 Volumina Ethanol auf 1 Volumen Wasser.

7. Verfahren nach Anspruch 6, **dadurch gekennzeichnet, dass** das Waschen in Schritt (d) einen ersten Waschschritt mit einem Ethanol/Wasser-Gemisch umfasst, wobei das Verhältnis des Gemisches 3 Volumina Ethanol auf ein Volumen Wasser beträgt.

8. Verfahren nach einem der vorhergehenden Ansprüche, **dadurch gekennzeichnet, dass** auf Schritt (c) eine Filtration des Reaktionsmediums über ein Filter mit einer Maschenweite von 0,5 bis 5 µm folgt, die vor Schritt (d) durchgeführt wird.

9. Verfahren nach Anspruch 8, **dadurch gekennzeichnet, dass** das eingesetzte Filter eine Maschenweite von 0,5 bis 1 µm aufweist.

10. Verfahren nach einem der vorhergehenden Ansprüche, **dadurch gekennzeichnet, dass** n = 0 bedeutet.

## Claims

1. Process for the industrial preparation of a disulphide of general formula **(I)** 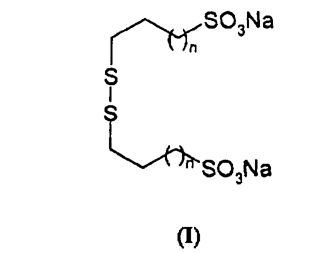 in which n represents an integer from 0 to 2,
**characterized in that** it comprises the following successive stages:
(a) treatment of a compound of general formula (II) 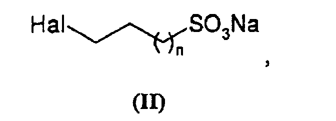 in which Hal is a halogen atom, with a thiolacetate of general formula CH₃COSM in which M represents a sodium or potassium atom;
(b) reaction of the thiolacetate obtained in stage (a) with a base followed by neutralization using an acid; and
(c) reaction of the sodium mercaptoalkylsulphonate of general formula (**III**) 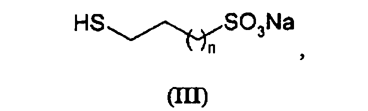 obtained in an intermediate manner, with oxygen, in order to produce the disulphide of general formula **(I)**;
(d) addition of ethanol to the reaction mixture obtained in stage (c), heating in order to solubilize the precipitates then cooling down and washing the solid obtained at least once with ethanol.

2. Process according to claim 1, **characterized in that** M is a sodium atom in stage (a).

3. Process according to claim 1 or 2, **characterized in that** the base is NaOH in stage (b).

4. Process according to one of claims 1 to 3, **characterized in that** the volume of ethanol added to stage (d) is comprised between 0.8 and 1.2 times the reaction volume obtained in stage (c).

5. Process according to claim 4, **characterized in that** the volume of ethanol added to stage (d) is comprised between 0.9 and 1.1 times the reaction volume obtained in stage (c).

6. Process according to one of the previous claims, **characterized in that** the washing of stage (d) comprises a first stage of washing with an ethanol/water mixture, the proportion of said mixture being greater than 2 volumes of ethanol for one volume of water.

7. Process according to claim 6, **characterized in that** the washing of stage (d) comprises a first stage of washing with an ethanol/water mixture, the proportion of said mixture being 3 volumes of ethanol for one volume of water.

8. Process according to one of the previous claims, **characterized in that** stage (c) is followed by filtration of the reaction medium on a filter with a mesh diameter comprised between 0.5 and 5 µm, which is carried out before stage (d).

9. Process according to claim 8, **characterized in that** the filter used has a mesh with a diameter comprised between 0.5 and 1 µm.

10. Process according to one of the previous claims, **characterized in that** n = 0.
